## Europäisches Patentamt
## European Patent Office
## Office européen des brevets

(11) Veröffentlichungsnummer: **0 366 856**
**A2**

# (12) EUROPÄISCHE PATENTANMELDUNG

(21) Anmeldenummer: **89106486.7**

(22) Anmeldetag: **12.04.89**

(51) Int. Cl.5: **A61B 17/00**

Die Bezeichnung der Erfindung wurde geändert (Richtlinien für die Prüfung im EPA, A-III, 7.3).

(30) Priorität: **06.10.88 DE 3833992**

(43) Veröffentlichungstag der Anmeldung: **09.05.90 Patentblatt 90/19**

(84) Benannte Vertragsstaaten: **AT BE CH DE ES FR GB GR IT LI LU NL SE**

(71) Anmelder: **Messerschmitt-Bölkow-Blohm Gesellschaft mit beschränkter Haftung Robert-Koch-Strasse D-8012 Ottobrunn(DE)**

(72) Erfinder: **Hessel, Stefan, Dr. Evastrasse 34 D-8000 München 81(DE)**

(54) **Lichtleiter-Bestrahlungseinrichtung.**

(57) Bei der Bestrahlungseinrichtung nach der Hauptanmeldung P 38 33 993.5, bei der ein schneidend wirkendes Laserlichtbündel (24) axial und ein koagulierend wirkendes Laserlichtbündel (34) schräg in eine einzige Lichtleitfaser (2) eingekoppelt werden derart, daß das erste Lichtbündel am distalen Lichtleitfaserende (28) mit axialer Abstrahlcharakteristik innerhalb eines zentralkegelförmigen Auskoppelwinkelbereichs (30) und das zweite Lichtbündel mit zirkumferenzieller Abstrahlcharakteristik innerhalb eines vom zentralkegelförmigen Auskoppelwinkelbereich getrennten und diesen ringkegelförmig umschließenden Auskoppelwinkelbereich (40) austritt, wird erfindungsgemäß in einem das distale Lichtleiterende aufnehmenden Gehäuseteil (6) eine beiden Auskoppelwinkelbereichen (30, 40) zugeordnete, optische Fokussiereinrichtung (10) angeordnet, durch welche die beiden Lichtbündel unter Beibehalt ihrer zueinander koaxialen, räumlichen Zuordnung auf einen außerhalb des Gehäuseteils liegenden Applikationsbereich (F) fokussiert werden.

FIG.1

## BESTRAHLUNGSEINRICHTUNG

Die Erfindung bezieht sich auf eine Bestrahlungseinrichtung, insbesondere zum kontaktlosen Schneiden und Koagulieren von biologischem Gewebe mittels optischer Strahlung hoher Intensität, nach dem Oberbegriff des Patentanspruchs 1.

Es ist bekannt, daß der therapeutische Effekt von Laser-Strahlen auf biologisches Gewebe wellenlängenabhängig ist. So läßt sich mit einer langwelligeren Laserstrahlung von z. B. 2,94 oder auch 1,32 μm eine vorwiegend schneidende Wirkung erzielen, während eine kurzwelligere Laserstrahlung z. B. die 1,06 μm Strahlung eines Neodym-Lasers eine vorwiegend koagulierende Wirkung besitzt. Bei dem aus der DE-OS 37 17 142 bekannten Bestrahlungsgerät werden die beiden Laserstrahlungen durch eine einzige, umschaltbare Laserlichtquelle erzeugt und nacheinander über ein und denselben Quarzlichtleiter zur Applikationsstelle übertragen. Häufig müssen jedoch im Operationsfeld beide Wirkungen gleichzeitig in räumlich voneinander abgegrenzten Teilzonen, nämlich die präzise, kontaktlose Schnittwirkung im Zentrum und der blutstillende Koagulationseffekt an den Randflächen der Schnittstelle zur Anwendung kommen, was bei diesem bekannten Gerät nicht möglich ist.

Bei dem aus der Hauptanmeldung P 38 33 993.5 bekannten Bestrahlungsgerät wird eine einzige Lichtleitfaser in der Weise mehrfach genutzt, daß zwei Laserlichtbündel verschiedener Wellenlänge in jeweils unterschiedlichen Einkoppel winkelbereichen so in die Lichtleitfaser eingestrahlt werden, daß das eine Lichtbündel am distalen Lichtleiterende mit axialer Abstrahlcharakteristik zentralkegelförmig und das andere Lichtbündel mit zirkumferenzieller Abstrahlcharakteristik in Form eines das zentralkegelförmige Lichtbündel umschließenden und zu diesem koaxialen Ringkegels austritt, wodurch auf baulich sehr einfache Weise eine selbsttätige Aufteilung der beiden Lichtbündel auf räumlich voneinander abgegrenzte Auskoppelwinkelbereiche am distalen Lichtleiterende gewährleistet wird und sich allein durch proximalseitige Steuereingriffe einerseits ein koagulierender und andererseits ein ablativer Laserlichteffekt wahlweise simultan oder zeitlich nacheinander jeweils individuell dosierbar an der Applikationsstelle erzielen läßt.

Erfindungsgemäß soll die Bestrahlungseinrichtung der eingangs genannten Art so ausgebildet werden, daß unter Beibehalt einer einzigen Lichtleitfaser eine räumlich getrennte, auf ein präzises, kontaktloses Schneiden von biologischem Gewebe bei gleichzeitiger Blutstillung abgestimmte Lichtbündelführung und -konzentration auf den Applikationsbereich garantiert wird, ohne daß es abstrahlseitig der Lichtleitfaser irgendwelcher selektiv wirkender Steuer- oder Trennorgane für die Lichtbündel bedarf.

Diese Aufgabe wird erfindungsgemäß durch die im Patentanspruch 1 gekennzeichnete Bestrahlungseinrichtung gelöst.

Aufbauend auf das Bestrahlungsgerät nach dem Hauptpatent, auf das hiermit voll inhaltlich Bezug genommen wird, wird erfindungsgemäß allein durch Anordnung einer baulich einfachen Fokussiereinrichtung abstrahlseitig des distalen Lichtleiterendes erreicht, daß die beiden, unabhängig voneinander steuerbaren Lichtbündel mit jeweils zu einem Bereich außerhalb des Gehäuseteils hin zunehmender Leistungsdichte derart abgelenkt werden, daß die schneidend wirkende, also etwa 2,94 μm Strahlung einen zum Applikationsbereich konvergierenden, zentralen Lichtkegel und die koagulierend wirkende, also etwa 1,06 μm Strahlung einen den Zentralkegel ringförmig umschließenden und zu diesem koaxialen Außenkegel bildet, wodurch mit äußerst geringem Bauaufwand eine für ein gleichzeitiges Schneiden und Koagulieren z. B. in der Parenchymchirurgie optimale Lichtbündelführung und -konzentration und eine problemlose, simultane Bedienung im Operationsfeld gewährleistet wird.

In weiterer vorteilhafter Ausgestaltung der Erfindung besitzen beide Lichtbündel gemäß Anspruch 2 einen gemeinsamen Fokuspunkt, wodurch es dem behandelnden Arzt möglich ist, allein durch Abstandsänderung des Gehäuseteils vom Operationsfeld beide Lichtbündel-Einzeleffekte gemeinsam zu verstärken oder abzuschwächen. Gemäß einem weiteren, bevorzugten Aspekt der Erfindung ist jedoch nach Anspruch 3 der Fokuspunkt des ringkegelförmig ausgekoppelten, koagulierend wirkenden Lichtbündels vom Gehäuseteil weiter entfernt als der Fokuspunkt des zentral ausgekoppelten, schneidend wirkenden Lichtbündels, so daß sich die beiden Lichtbündel-Einzeleffekte wiederum allein durch Abstandsänderungen des Gehäuseteils vom Operationsfeld und ohne weitere Steuereingriffe noch erheblich besser dosieren lassen, derart, daß sich der eine Einzeleffekt verstärkt und gleichzeitig der andere abschwächt, wenn das Operationsfeld relativ zum Gehäuseteil im Bereich zwischen den Fokuspunkten der Lichtbündel bewegt wird. Eine baulich besonders einfache Ausbildung der Fokussiereinrichtung mit voneinander beabstandeten Fokuspunkten wird zweckmäßigerweise dadurch erreicht, daß als Fokussiereinrichtung gemäß Anspruch 4 ein asphärisches Linsensystem bzw. gemäß Anspruch 5 für Lichtbündel unterschiedlicher Wellenlängen ein von beiden Lichtbündeln durchstrahltes Linsensystem aus einem Material hoher Farbdispersion vorgesehen ist. Aus Handhabungsgründen ist das Gehäuseteil gemäß Anspruch 6 vorzugsweise als Fokussierhandstück ausgebildet.

Abgesehen von den erwähnten Abstandsänderungen sind die unterschiedlichen Einzeleffekte der Lichtbündel gemäß Anspruch 7 vorzugsweise auch jeweils individuell dadurch einstellbar, daß die Lichtbündel hinsichtlich Strahlungsleistung, Wellenlänge und/oder Betriebsart verschiedenartig bzw. unabhängig voneinander steuerbar sind.

Zur Erzeugung der beiden Lichtbündel in Form von Laserlichtbündeln unterschiedlicher Wellenlänge mit jeweils individuell dosierbarer Intensität sind gemäß Anspruch 8 vorzugsweise zwei getrennte Laserlichtquellen mit nachgeschalteter, einkoppelseitiger Optik oder in weiterer baulicher Vereinfachung gemäß Anspruch 9 eine einzige, entsprechend der gewünschten, individuellen Dosierung wellenlängenumschaltbare Laserlichtquelle mit einem nachgeschalteten, die Laserstrahlung wellenlängenabhängig auf den zugeordneten Einkoppelwinkelbereich ablenkenden Prisma vorgesehen, wobei zum selektiven Ein- und Ausblenden jedes Lichtbündels gemäß Anspruch 10 vorzugsweise ein umschaltbares, optisches Element im Strahlengang der einkoppelseitigen Optik angeordnet ist, wodurch sich auf baulich einfache Weise auch jeder der beiden Einzeleffekte für sich allein zur Anwendung bringen läßt.

Die Erfindung wird nunmehr anhand der in den Fig. schematisch dargestellten Ausführungsbeispiele näher beschrieben. Es zeigen:

Fig. 1 eine Bestrahlungseinrichtung zum gleichzeiti gen Schneiden (a) und Koagulieren (b) von biologischem Gewebe; und

Fig. 2 die Bestrahlungseinrichtung gemäß Fig. 1 mit einer modifizierten Laserlichtquelle und einem abgewandelten Linsensystem.

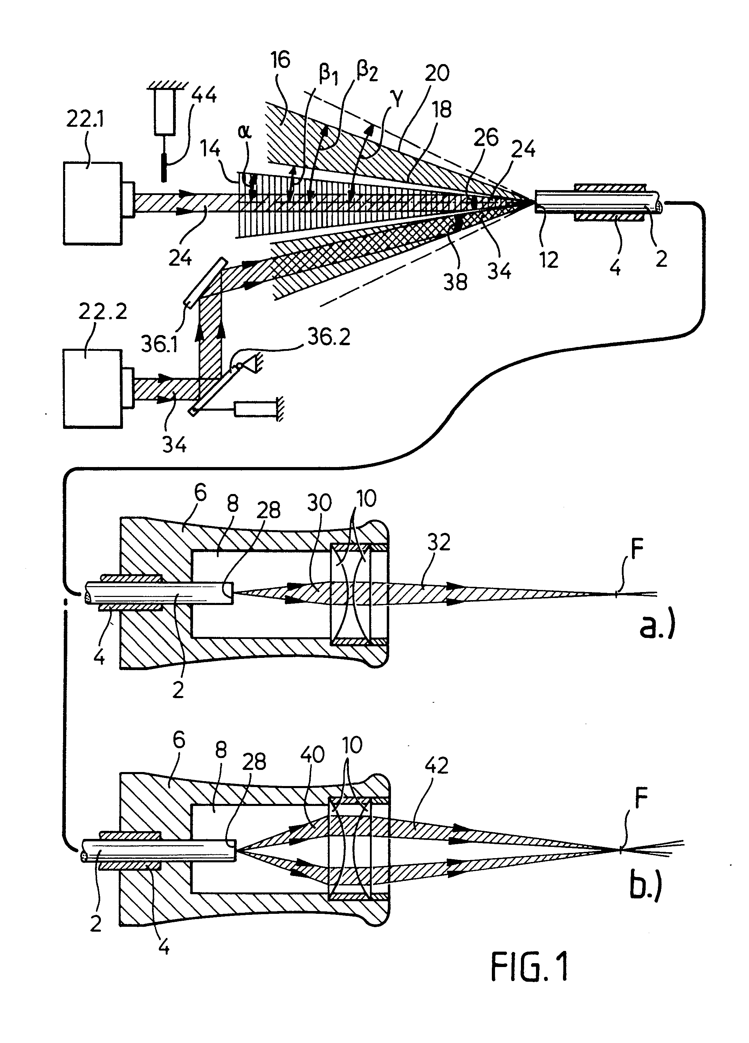

Gemäß Fig. 1 enthält die Bestrahlungseinrichtung eine flexible Multimode-Lichtleitfaser 2 einschließlich einer Faserhülle 4, sowie ein am distalen Lichtleiterende angeordnetes Gehäuseteil 6 in Form eines Fokussierhandstücks mit einem zentralen, das distale Lichtleiterende aufnehmenden Hohlraum 8, in dem sich eine als Linsensystem ausgebildete Fokussiereinrichtung 10 befindet.

Auf der senkrecht zur Faser-Längsachse verlaufenden Einkoppel-Stirnfläche 12 sind der Lichtleitfaser 2 innerhalb des in Fig. 1 gestrichelt gezeichneten Akzeptanzkegels (Akzeptanzwinkel $\gamma$) zwei unterschiedliche Einkoppelwinkelbereiche 14 und 16 zugeordnet, von denen der eine - 14 - durch einen zur Faserlängsachse koaxialen Innenkegel mit dem halben Scheitelwinkel $\alpha$ und der zweite - 16 - durch einen zum Innenkegel 14 koaxialen und diesen ringförmig umschließenden Außenkegel gebildet wird, welch letzterer von zwei Kegelmantelflächen 18 und 20, die einen gegenüber dem Winkel $\alpha$ stufenweise erhöhten Öffnungswinkel $\beta_1$ bzw. $\beta_2$ besitzen begrenzt wird.

Der schneidende Effekt der Bestrahlungseinrichtung wird durch ein von einer Laserlichtquelle 22.1 emittiertes Lichtbündel 24 erzeugt, das eine hauptsächlich ablative, vaporisierende Wirkung auf biologisches Gewebe, also eine Wellenlänge von z. b. 2,94 $\mu$m besitzt und durch eine Fokussierlinse 26 innerhalb des Einkoppelwinkelbereichs 14 auf die Lichtfaser-Stirnfläche 12 fokussiert wird, so daß es an der distalen, ebenfalls senkrecht zur Lichtfaser-Längsachse verlaufenden Faser-Stirnfläche 28 mit axialer Abstrahlcharakteristik innerhalb eines dem Einkoppelwinkelbereich 14 geometrisch entsprechenden, zentralkegelförmigen Auskoppelwinkelbereichs 30 in den Hohlraum 8 austritt und durch das Sammellinsensystem 10 in Form eines in Strahlungsrichtung konvergenten, zentralen Lichtkegels 32 auf einen außerhalb des Gehäuseteils 6 liegenden Fokuspunkt F fokussiert wird (Fig. 1a).

Um zusätzlich zum schneidenden auch zugleich einen blutstillenden Effekt durch Gewebekoagulation im Operationsfeld zu erzielen, ist eine zweite Laserlichtquelle 22.2 vorgesehen, die ein hauptsächlich koagulierend wirkendes Lichtbündel 34 mit einer Wellenlänge von z. B. 1,06 $\mu$m erzeugt, welches über ein Spiegelsystem 36 und eine Fokussierlinse 38 in Form eines innerhalb des zweiten Einkoppelwinkelbereichs 16 liegenden Lichtkegels auf die Lichtfaser-Stirnfläche 12 fokussiert wird. Aufgrund der andersartigen Einkoppelung tritt dieses Lichtbündel 34 am distalen Lichtfaserende mit zirkumferenzieller Abstrahlcharakteristik in einem dem Einkopelwinkelbereich 16 bezüglich der Flächennormalen der Lichtfaser-Stirnflächen 12 bzw. 28 geometrisch entsprechenden Auskoppelwinkelbereich 40, also in Form des in Fig. 1b kreuzschraffierten Ringkegels, in den Hohlraum 8 aus, derart, daß es das Sammellinsensystem 10 radial außerhalb des zentralen Lichtbündels 24 durchdringt und dabei ebenfalls auf den Fokuspunkt F fokussiert wird, so daß es einen zum schneidend wirkenden Zentralkegel 32 koaxialen und diesen ringförmig umschließenden Außenkegel 42 mit zum Fokalbereich F hin zunehmender Koagulationswirkung bildet.

Aufgrund der besonderen, räumlichen Zuordnung der beiden Laserlichtkegel 32, 42 mit Hilfe des für beide Wellenlängen transparenten, fokussierenden Linsensystems 10 wird somit die schneidend wirkende 2,94 $\mu$m Laserstrahlung auf das Zentrum des Operationsfeldes gelenkt, während die 1,06 $\mu$m Strahlung den umliegenden Randbereich koaguliert, so daß ein präzises kontaktloses Schneiden von biologischem Gewebe mit der zentralen Laserstrahlung 24 bzw. 32 bei gleichzeitiger Blutstillung durch Koagulation der randseitigen Schnittflächen mit dem Laserstrahlbündel 34 bzw. 42 möglich ist.

Durch eine Änderung des Abstands des Gehäuseteils 6 vom Operationsfeld können der Koagulations- und der AblationsEffekt gemeinsam variiert werden. Zur individuellen Dosierung der beiden Laserlichteffekte unabhängig voneinander sind die Laserlichtquellen 22.1 und 22.2 hinsichtlich der Betriebsparamenter, z. B. der Strahlungsleistung, der Betrahlungsdauer oder der Betriebsart jeweils einzeln steuerbar. Eine wechselweise Umschaltung von dem einen auf den anderen Laserlichteffekt läßt sich nicht nur durch entsprechende Steuerung der beiden Lichtquellen 22, sondern auch durch umschaltbare, im Strahlengang der Laserlichtbündel 24 und 34 angeordnete, optische Elemente erreichen, also etwa eine in den Strahlengang des Lichtbündels 24 einfahrbare Blende 44 oder den umklappbaren Spiegel 36.2 des Spiegelsystems 36.

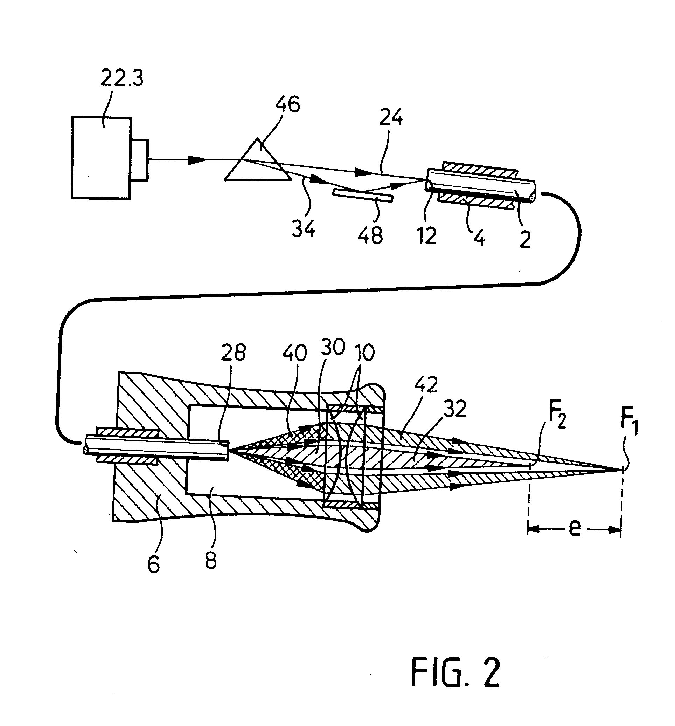

Die in Fig. 2 gezeigte Bestrahlungseinrichtung unterscheidet sich von dem ersten Ausführungsbeispiel im Wesentlichen nur durch eine andere Art des einkoppelseitigen Lichterzeugungssystems sowie dadurch, daß die Lichtbündel unterschiedliche Fokuspunkte besitzen. Die dem ersten Ausführungsbeispiel entsprechenden Bauelemente sind daher durch das gleiche Bezugszeichen gekennzeichnet. Bei dem Bestrahlungssystem gemäß Fig. 2 ist nur eine einzige Lichtquelle 22.3 vorhanden, etwa in Form eines Neodym-YAG-Lasers, der zwischen zwei unterschiedlichen Wellenlängen, nämlich einer hauptsächlich schneidend wirkenden Laserstrahlung von z. B. 1,32 μm und einer hauptsächlich koagulierend wirkenden Laserstrahlung von z. B. 1,06 μm, umschaltbar ist. Durch ein dem Laser 22.3 nachgeschaltetes Prisma 46 wird die Laserstrahlung wellenlängenabhängig abgelenkt, so daß das langwelligere Laser-Lichtbündel 24 in der der Fig. 1 entsprechenden Weise zentral in die Faser-Stirnfläche 12 eingekoppelt wird und an dem distalen Ende des Gehäuseteils 6 in Form eines schneidend wirkenden Zentralkegels 32 austritt, während die am Prisma 46 stärker abgelenkte, kurzwelligere Laserstrahlung über einen Spiegel 48 innerhalb des Einkoppelwinkelbereichs 16 gemäß Fig. 1 schräg in die Lichtleitfaser 2 eingekoppelt und am distalen Ende des Gehäuseteils 6 in Form eines den Zentralkegel 32 ringförmig umschließenden Außenkegels 42 abgestrahlt wird. Durch Verwendung von asphärischen Linsen 10 oder durch gezielte Ausnutzung der chromatischen Linsenfehler mit optischen Materialien hoher Farbdispersion für das Linsensystem 10 wird der Fokuspunkt $F_1$ des Außenkegels 42 in Strahlungsrichtung vor den Fokuspunkt $F_2$

des Zentralkegels 32 gelegt. Somit können durch einfache Variation des Arbeitsabstandes des Gehäuseteils 6 vom Operationsfeld innerhalb des Fokusabstandes e die schneidende Wirkung des zentralen Lichtbündels 32 und die koagulierende Wirkung des außenliegenden Lichtbündels 42 gegensinnig zueinander selektiv verändert werden.

Im übrigen ist die Bau- und Betriebsweise der in Fig. 2 gezeigten Bestrahlungseinrichtung die gleiche wie bei dem ersten Auführungsbeispiel.

## Ansprüche

1. Bestrahlungseinrichtung, insbesondere zum kontaktlosen Schneiden und Koagulieren von biologischem Gewebe, mittels optischer Strahlung hoher Intensität, bei der zur Übertragung zweier voneinander unabhängiger Lichtbündel eine einzige gemeinsame Lichtleitfaser vorgesehen ist, in die die beiden Lichtbündel eintrittseitig in jeweils unterschiedlichen Einkoppelwinkelbereichen eingestrahlt werden derart, daß das eine Lichtbündel am distalen Lichtleitfaserende mit axialer Abstrahlcharakteristik innerhalb eines ersten, zur Faser-Längsachse zentralkegelförmigen Auskoppelwinkelbereichs und das andere Lichtbündel mit zirkumferenzieller Abstrahlcharakteristik innerhalb eines vom ersten getrennten und diesen ringkegelförmig umschließenden, zweiten Auskoppelwinkelbereichs austritt, wobei für das zentral ausgekoppelte Lichtbündel eine vorwiegend schneidende und für das ringkegelförmig ausgekoppelte eine vorwiegend koagulierende Licht-, insbesondere Laserlichtstrahlung vorgesehen ist, nach der Deutschen Patentanmeldung P38 33 993.5,
dadurch **gekennzeichnet,** daß in dem
das distale Lichtleiterende aufnehmenden Gehäuseteil (6) eine beiden Auskoppelwinkelbereichen (30, 40) zugeordnete, optische Fokussiereinrichtung (10) angeordnet ist, durch welche die beiden Lichtbündel (24, 34) unter Beibehalt ihrer zueinander koaxialen, räumlichen Zuordnung auf einen außerhalb des Gehäuseteils liegenden Applikationsbereich (F, $F_1$, $F_2$) fokussiert werden.

2. Bestrahlungseinrichtung nach Anspruch 1, dadurch **gekennzeichnet,** daß
beide Lichtbündel (24, 34) einen gemeinsamen Fokuspunkt (F) im Applikationsbereich besitzen.

3. Bestrahlungseinrichtung nach Anspruch 1, dadurch **gekennzeichnet,** daß
die Lichtbündel (24, 34) in Richtung ihrer gemeinsamen Strahlungsachse hintereinanderliegende Fokuspunkte ($F_1$, $F_2$) besitzen, wobei der Fokuspunkt ($F_1$) des ringkegelförmig ausgekoppelten Lichtbündels (34) vom Gehäuseteil (6) weiter entfernt ist als der Fokuspunkt ($F_2$) des zentral ausgekoppelten Lichtbündels (24).

4. Bestrahlungseinrichtung nach Anspruch 3, dadurch **gekennzeichnet,** daß als Fokussiereinrichtung (10) ein von beiden Lichtbündeln (24, 34) durchstrahltes, aphärisches Linsensystem vorgesehen ist.

5. Bestrahlungeinrichtung nach Anspruch 3, dadurch **gekennzeichnet,** daß für Lichtbündel (24, 34) unterschiedlicher Wellenlänge als Fokussiereinrichtung (10) ein von beiden Lichtbündeln durchstrahltes Linsensystem aus einem Material hoher Farbdispersion vorgesehen ist.

6. Bestrahlungseinrichtung nach einem der vorhergehenden Ansprüche, dadurch **gekennzeichnet,** daß das Gehäuseteil (6) als Fokussierhandstück ausgebildet ist.

7. Bestrahlungseinrichtung nach einem der vorhergehenden Ansprüche, dadurch **gekennzeichnet,** daß die Lichtbündel (24, 34) hinsichtlich Strahlungsleistung, Wellenlänge und/oder Betriebsart verschiedenartig bzw. unabhängig voneinander steuerbar sind.

8. Bestrahlungseinrichtung nach einem der vorhergehenden Ansprüche, dadurch **gekennzeichnet,** daß zur gleichzeitigen Erzeugung beider Lichtbündel (24, 34) zwei getrennte Laserlichtquellen (22.1, 22.2) mit unterschiedlichen Wellenlängen und einer einkoppelseitigen Optik (26, 36, 38) zur Einkopplung der Lichtbündel innerhalb des jeweils vorgegebenen Einkoppelwinkelbereichs (14 bzw. 16) vorgesehen sind.

9. Bestrahlungseinrichtung nach einem der Ansprüche 1 bis 7, dadurch **gekennzeichnet,** daß eine einzige Laserlichtquelle (22.3) zur Erzeugung der beiden Lichtbündel (24, 34) mit unterschiedlichen Wellenlängen und eine einkoppelseitige Optik (46, 48) einschließlich eines die Lichtbündel wellenlängenabhängig ablenkenden Prismas (46) vorgesehen sind.

10. Bestrahlungseinrichtung nach einem der vorhergehenden Ansprüche, dadurch **gekennzeichnet,** daß die einkoppelseitige Optik mindestens ein umschaltbares optisches Element (36.2, 44) zum selektiven Ein- und Ausblenden zumindest eines der Lichtbündel (24, 34) enthält.

FIG.1

FIG. 2